# EUROPEAN PATENT APPLICATION

(11) **EP 3 572 658 A1**
(43) Date of publication of application: **27.11.2019**
(21) Application number: 19461533.2
(22) Date of filing: 07.05.2019
(51) Int. Cl.: F02F 3/28, F01B 9/02, F02B 75/32, F16J 1/24, F02B 75/02

(54) **INTERNAL COMBUSTION ENGINE, IN PARTICULAR TWO-STROKE ENGINE**

(30) Priority: 24.05.2018 PL 42572418
(71) Applicant: 3D Gence Spólka Z Ograniczona Odpowiedzialnoscia, 40-085 Katowice (PL)
(72) Inventor: Wolnicki, Przemysaw, 42-100 Klobuck (PL); Kostrzewa, Szymon, 05-622 Belsk Duzy (PL)
(74) Representative: AOMB Polska Sp. z.o.o.

(57) **Abstract**

The present invention relates to an internal combustion engine, especially two-stroke engine, powered by an air-fuel mixture. The engine comprises means for forcing a resultant motion of the piston (2) comprising simultaneous reciprocating and rotary motions, wherein the geometry of a bottom of the piston (2) and the arrangement of the openings in the cylinder (1) are such that the piston (2) travelling in the cylinder (1) opens and closes alternately the at least one suction opening (3) and the at least one exhaust opening (3').

## Description

### Technical field

The present invention relates to an internal combustion engine, especially two-stroke, powered by an air-fuel mixture.

### Background art

Known from the prior art two stroke engine comprises a cylinder with openings for the mixture inlet and the exhaust gas outlet and a piston slidably placed therein, mounted in a self-aligning arrangement on a connecting-rod linked with another end to a crankshaft. This engine has a two-stroke working cycle, i.e. for every crankshaft rotation. In the compression stroke, the cylinder is washed by the air-fuel mixture.

A disadvantage of the known engine is the mixing of the exhaust gas with the air-fuel mixture in the compression stroke, which mixture partially non-combusted escapes into the exhaust system. As a result, the engine has a high fuel consumption and low efficiency, and the level of pollutants emitted with the exhaust gas is high.

Another known two-stroke crosshead engine has a piston rigidly mounted on the piston rod. The crankshaft is connected to the piston rod by a crosshead.

### Summary of invention

The objective of the present invention is to provide an engine that is free from mixing of the exhaust gas with the air-fuel mixture, environment friendly and cost effective.

According to the invention, this objective is achieved by an internal combustion engine, especially two-stroke engine, comprising at least one cylinder with at least one suction opening for the air-fuel mixture inlet and at least one exhaust gas opening for the exhaust gas outlet, and a piston placed in the cylinder connected rigidly to a piston rod. The engine further comprises means for forcing a resultant motion of the piston comprising simultaneous reciprocating and rotary motions, wherein the geometry of the a bottom of the piston and the arrangement of the openings in the cylinder are such that the piston travelling in the cylinder opens and closes alternately the at least one suction opening and the at least one exhaust opening.

The means for forcing the resultant motion of the piston preferably comprises an intersecting axis gear coupled to an engine drive shaft and a slidably axially bearing-mounted piston rod.

In one preferred embodiment, the intersecting axis gear is a bevel gear with the first gear meshed with the second gear. The piston rod is slidably axially bearing-mounted in the first gear rotating in the axis of the piston and coupled thereto by a form-fitting connection, wherein the crank transferring the reciprocating motion of the piston to the second gear rigidly fixed to the engine drive shaft is pivotally attached to the piston rod.

The bottom of the piston is preferably oblique.

### Brief description of drawings

The invention will now be described by a way of example only with reference
to the accompanying drawings, in which:
Fig. 1 shows the engine in an oblique projection,
Fig. 2 shows the engine in cross section through the piston and cylinder in the position wherein the exhaust gas is removed,
Fig. 3 shows the engine in cross section through the piston and cylinder in the position wherein the air-fuel mixture is taken in the cylinder,
Fig. 4 shows the engine in cross section through the piston and cylinder in the position wherein the air-fuel mixture is compressed.

### Detailed description of preferred embodiments of the invention

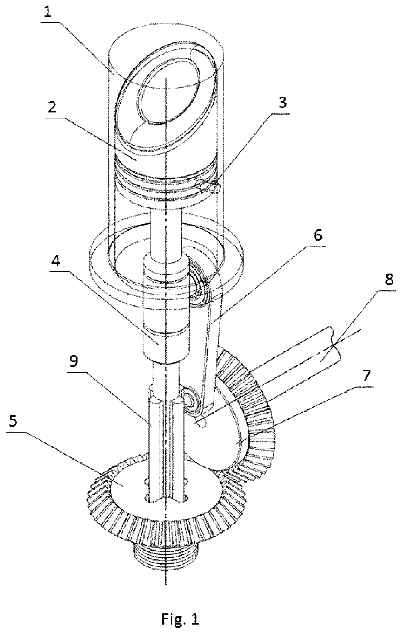

In one of the preferred embodiments of the invention, Fig. 1 shows schematically a two-stroke combustion engine comprising at least one cylinder 1 with a suction opening 3 for the air-fuel mixture inlet and an exhaust opening 3' for the exhaust gas outlet. In the cylinder 1 there is a movably placed piston 2 with an oblique ellipse-shaped bottom at the top, obtained by cutting the piston 2 with a plane.

Although in the preferred embodiment shown in the drawing the piston 2 has an oblique bottom, the use of a bottom of another shape is also possible, as long as the bottom geometry and the arrangement of the openings 3, 3' in the cylinder 1 are such that the piston 2 travelling in the cylinder 1 will open and close alternately the suction opening 3 and the exhaust opening 3'. For example, it can be a stepped bottom from which the half of the cylinder has been cut. Essentially, the condition is fulfilled by any bottom whose surface, as a whole, is not perpendicular to the axis of the piston 2, i.e. such a piston 2, which has at least two parts with different wall heights.

The engine comprises means for forcing a resultant motion of the piston 2, i.e. the simultaneous reciprocating and rotary motions. These means comprise an intersecting axis gear coupled to an engine drive shaft 8 and a slidably axially bearing-mounted piston rod 9. In the present example, the intersecting axis gear is a bevel gear comprising the first gear 5 meshed with the second gear 7.

In order to force the resultant motion, the rigid piston rod 9 is attached to the piston 2 at the bottom. The piston rod 9 is axially and rigidly fixed in a sleeve 4. The sleeve 4 has a mandrel protruding perpendicularly to its axis, on which one end of a crank 6 is bearing-mounted, whereas the other end of the crank 6 is bearing-mounted on the pin rotating the second gear 7, rigidly attached to the engine drive shaft 8. The crank 6 converts the reciprocating motion of the piston 2 to the rotary motion of the second gear 7. The second gear 7 of the drive shaft 8 is mated with the first gear 5 mounted coaxially on the piston rod 9, slidably through a form-fitting connection. Both gears 5 and 7 form a bevel gear with oblique teeth. The form-fitting connection of the horizontal gear 5 with the piston rod 9 ensures the rotary motion of the piston 2.

The suction opening 3 and the exhaust opening 3' are arranged circumferentially on the cylinder 1 closer to the lower extreme position of the piston 2, preferably symmetrically with respect to the axis of the piston 2. In the example shown in the drawing, the suction opening 3 and the exhaust opening 3' have an elongated (bean-like) shape, corresponding to the elliptical shape of the edge of the bottom. Other shapes of the openings are also possible, e.g. round, perpendicular or parallel to the piston axis, etc.

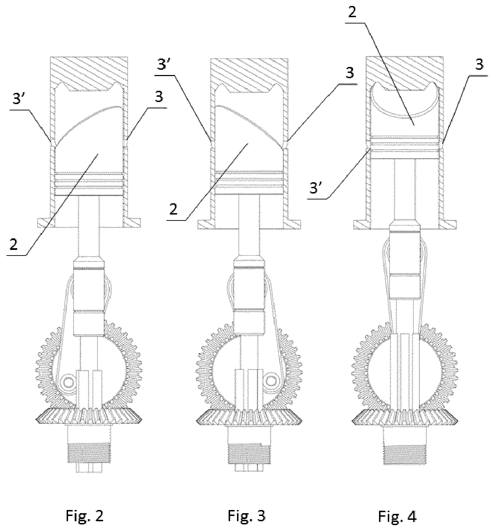

Fig. 2-4 show the operation cycle of a two-stroke engine according to the invention. During the engine operation, the force generated by the combustion of the air-fuel mixture acts on the piston 2 and the piston rod 9 connected thereto by means of the sleeve 4, forcing the reciprocating motion. Through the crank 6 and the second gear 7 the force is transmitted to the engine drive shaft 8. Meshed with the second gear 7, the first gear 5 forces the piston rod 9 and the piston 2 to rotate. During the power-exhaust stroke (Fig. 2), when the piston 2 moves from the upper extreme position due to the explosion of the air-fuel mixture to the lower extreme position, in the final phase of this stroke, the simultaneous reciprocating and rotary motions of the piston 2 result in opening the window of the exhaust opening 3' and at the same time closing the window of the suction opening 3 - the piston 2 is then positioned such that the part of the piston 2 with the lower wall oriented towards the exhaust opening 3' uncovers it and the part of the piston 2 with the higher wall is at the same time conceals the suction opening 3. Opening of the exhaust opening 3' occurs when the piston 2 is near the lower extreme position, whereas the opening 3' can remain open until the piston 2 reaches the lower extreme position or only until just before it reaches the lower extreme position - which means that in the lower extreme position the exhaust opening 3' is closed (by the way, the suction opening 3 is then also closed).

In the course of the suction-compression stroke (Fig. 3), when the piston 2 moves from the lower extreme position to the upper extreme position (Fig. 4), in the first phase of this stroke, the simultaneous reciprocating and rotary motions of the piston 2 result in opening the window of the suction opening 3 and closing the window of the exhaust opening 3' - the piston 2 is then set in a position rotated 180 degrees with respect to the position it occupied during the power-exhaust stroke (in Fig. 2), i.e. the part of the piston 2 with the lower wall uncovers now the suction opening 3 and the part of the piston 2 with the higher wall simultaneously conceals the exhaust opening 3'. The opening of the suction opening 3 occurs when the piston 2 is near the lower extreme position, whereas the opening 3 can remain open when the piston 2 is in the lower extreme position or just after it has moved up, but is still close to the lower extreme position. Then, after filling the cylinder 1 with the air-fuel mixture, the further movement of the piston 2 up results in closing both openings 3 and 3', compression of the mixture, explosion and the cycle is repeated as described above.

In the engine according to the invention, during the operating cycle there is no mixing of the exhaust gas with the air-fuel mixture. There is also no scavenge, i.e. entering of the air-fuel mixture into the outlet opening.

An additional advantage of the engine according to the invention is that it does not have a timing gear system. The synchronisation of the engine's operation is forced by its design.

Another advantage of the invention is that due to the forced rotary motion of the piston inside the combustion chamber of the cylinder turbulences are produced which are advantageous for the air-fuel mixture to mix.

## Claims

1. An internal combustion engine, especially two-stroke one, comprising at least one cylinder (1) with at least one suction opening (3) for the air-fuel mixture inlet and at least one exhaust gas opening (3') for the exhaust gas outlet, and a piston (2) placed in the cylinder (1) connected rigidly to a piston rod (9), **characterised in that** it comprises means for forcing a resultant motion of the piston (2) comprising simultaneous reciprocating and rotary motions, wherein the geometry of a bottom of the piston (2) and the arrangement of the openings in the cylinder (1) are such that the piston (2) travelling in the cylinder (1) opens and closes alternately the at least one suction opening (3) and the at least one exhaust opening (3').

2. The engine according to claim 1, **wherein** the means for forcing the resultant motion of the piston (2) comprise an intersecting axis gear coupled to an engine drive shaft (8) and a slidably axially bearing-mounted piston rod (9).

3. The engine according to claim 2, **wherein** the intersecting axis gear is a bevel gear with the first gear (5) meshed with the second gear (7).

4. The engine according to claim 3, **wherein** the piston rod (9) is slidably axially bearing-mounted in the first gear (5) rotating in the axis of the piston (2) and coupled thereto by a form-fitting connection, wherein the crank (6) transferring the reciprocating motion of the piston (2) to the second gear (7) rigidly fixed to the engine drive shaft (8) is pivotally attached to the piston rod (9).

5. The engine according to any preceding claim 1 to 4, **wherein** the bottom of the piston (2) is oblique.
